# EUROPEAN PATENT APPLICATION

(11) **EP 4 555 914 A1**
(43) Date of publication of application: **21.05.2025**
(21) Application number: 24176980.1
(22) Date of filing: 21.05.2024
(51) Int. Cl.: A61B 1/00, A61B 1/05, G02B 23/24, H04N 23/50

(54) **PICKUP UNIT, ENDOSCOPE, AND METHOD FOR MANUFACTURING IMAGE PICKUP UNIT**

(30) Priority: 15.11.2023 US 202363599284 P
(71) Applicant: Olympus Medical Systems Corp., Tokyo 192-8507 (JP)
(72) Inventor: ASAKURA, Yo, Tokyo, 192-8507 (JP); EGUCHI, Yosuke, Tokyo, 192-8507 (JP)
(74) Representative: Schicker, Silvia

(57) **Abstract**

An image pickup unit includes a three-dimensional wiring board including a recessed portion having a side surface and a bottom surface; a camera module disposed in the recessed portion; and resin having a light shielding property that fills a gap formed between the side surface and the camera module and between the bottom surface and the camera module. In the resin, a transmittance and a reflectance of light having a wavelength of 380 to 780 nm are equal to or lower than 0.5% and equal to or lower than 5%, respectively, and when a value of a bidirectional reflectance distribution function in a direction in which an azimuth angle from an incident surface is ϕr and a polar angle is θr, with a polar angle in an incident direction as θi is BRDF (θi, ϕr, θr), in a case where θi is 45 to 75 degrees, ϕr is -60 to 60 degrees, and θr is -85 to 85 degrees, the value of the bidirectional reflectance distribution function is equal to or lower than 0.1 of incident light, in which, ϕr ≠ 0 and θi ≠ θr.

## Description

### FIELD

The present invention relates to an image pickup unit including resin having a light shielding property, an endoscope, and a method for manufacturing the image pickup unit.

### BACKGROUND

Conventionally, an image pickup unit has been disposed in a distal end portion of an endoscope. The image pickup unit includes a camera unit in a recessed portion provided in a three-dimensional wiring board. A portion between the recessed portion and the camera unit is filled with light shielding resin for the purpose of improving bonding strength, water tightness, and a light shielding property.

For example, Japanese Patent Application Laid-Open Publication No. 2012-189788 discloses a camera module in which a gap between a main body of a camera module and a shield is filled with resin having a light shielding property formed in black by adding a black pigment of carbon black.

### SUMMARY OF THE INVENTION

An image pickup unit of one aspect of the present invention includes: a three-dimensional wiring board including a recessed portion having a side surface and a bottom surface; a camera module disposed in the recessed portion; and resin having a light shielding property that fills a gap formed between the side surface and the camera module and between the bottom surface and the camera module, in which in the resin, a transmittance and a reflectance of light having a wavelength of 380 to 780 nm are equal to or lower than 0.5% and equal to or lower than 5%, respectively, and when a value of a bidirectional reflectance distribution function in a direction in which an azimuth angle from an incident surface is ϕr and a polar angle is θr, with a polar angle in an incident direction as θi is BRDF (θi, ϕr, θr), in a case where θi is 45 to 75 degrees, ϕr is -60 to 60 degrees, and θr is -85 to 85 degrees, the value of the bidirectional reflectance distribution function is equal to or lower than 0.1 of incident light, wherein, ϕr ≠ 0 and θi ≠ θr.

An endoscope of one aspect of the present invention includes an image pickup unit at a distal end of an insertion portion inserted into a subject, the image pickup unit including: a three-dimensional wiring board including a recessed portion having a side surface and a bottom surface; a camera module disposed in the recessed portion; and resin having a light shielding property that fills a gap formed between the side surface and the camera module and between the bottom surface and the camera module, in which in the resin, a transmittance and a reflectance of light having a wavelength of 380 to 780 nm are equal to or lower than 0.5% and equal to or lower than 5%, respectively, and when a value of a bidirectional reflectance distribution function in a direction in which an azimuth angle from an incident surface is ϕr and a polar angle is θr, with a polar angle in an incident direction as θi is BRDF (θi, ϕr, θr), in a case where θi is 45 to 75 degrees, ϕr is -60 to 60 degrees, and θr is -85 to 85 degrees, the value of the bidirectional reflectance distribution function is equal to or lower than 0.1 of incident light, where ϕr ≠ 0 and θi ≠ θr.

A method for manufacturing of an image pickup unit of one aspect of the present invention includes steps of: connecting wiring of a three-dimensional wiring board and a camera module via an external electrode, with the camera module housed in a recessed portion of the three-dimensional wiring board; filling, with first resin, a gap between the recessed portion of the three-dimensional wiring board and the camera module, from a bottom surface of the recessed portion to a position of an aperture of the camera module; and filing, with second resin, a gap between the recessed portion of the three-dimensional wiring board and the camera module, from the aperture to an outermost surface of a stacked lens of the camera module, in which the first resin is a resin having a greater dielectric breakdown resistance value and a smaller viscosity than the second resin.

### BRIEF DESCRIPTION OF THE DRAWINGS

Fig. 1 is an overall configurational view showing an example of an overall configuration of an endoscope according to a first embodiment;
Fig. 2 is a perspective view showing a configuration of an image pickup unit of the first embodiment;
Fig. 3 is a cross-sectional view taken along line III-III of Fig. 2;
Fig. 4 is a view for explaining a relation between incident light and reflected light; and
Fig. 5 is a cross-sectional view showing a configuration of an image pickup unit of a second embodiment.

### DETAILED DESCRIPTION

In general, there have been cases in which even when a gap between a main body of a camera module and a shield is filled with resin having a light shielding property, scattered light on a surface cannot be reduced, and thus, flare is generated.

According to embodiments described below, it is possible to provide an image pickup unit, an endoscope, and a method for manufacturing the image pickup unit that are capable of reducing scattered light on a surface to suppress generation of flare.

Hereinafter, the embodiments will be described in detail with reference to the drawings.

Note that the drawings based on the embodiments are schematic illustrations and the relations in thickness and width among the portions, the ratios in thickness and the relative angles among the portions, and the like are different from the actual relations in thickness and width, ratios in thickness, and relative angles. Portions having different relations and ratios in dimension among the drawings are included. A direction in which light is made incident is referred to as "up."

### (First embodiment)

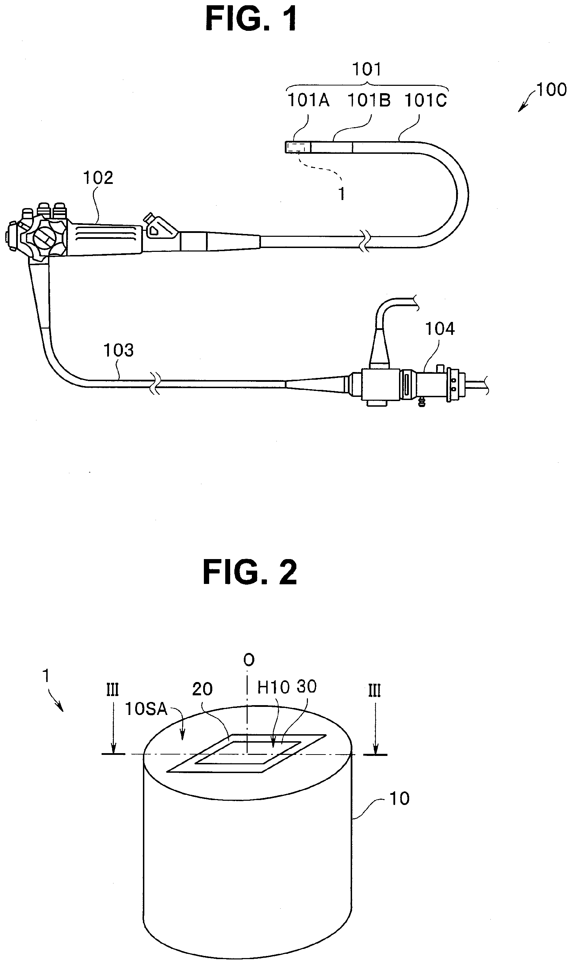

Fig. 1 is an overall configurational view showing an example of an overall configuration of an endoscope according to a first embodiment.

As shown in Fig. 1, an endoscope 100 includes an insertion portion 101, an operation portion 102, a universal cord 103, and an endoscope connector 104.

The insertion portion 101 in an elongated tubular shape is inserted into a body cavity of a living body. The insertion portion 101 includes a distal end portion 101A, a bending portion 101B, and a flexible tube 101C that are continuously provided in sequence from a distal end side, and has flexibility as a whole.

The distal end portion 101A includes an image pickup unit 1 for acquiring image information on a subject. The distal end portion 101A includes a treatment instrument insertion channel, an illumination unit, and the like in addition to the image pickup unit 1.

The bending portion 101B bends in up-down and left-right directions in accordance with rotating operation of a bending knob of the operation portion 102 for performing bending operation.

The flexible tube 101C is a flexible tubular member that is passively flexible. The treatment instrument insertion channel, various electric signal lines, a light guide fiber bundle, and the like are inserted through the inside of the flexible tube 101C. The electric signal lines extend from the image pickup unit 1 that is built in the distal end portion 101A through the operation portion 102 to the universal cord 103. The light guide fiber bundle guides the light from a light source apparatus that is external equipment to a distal end surface of the distal end portion 101A.

The operation portion 102 is provided continuously with a proximal end portion of the insertion portion 101, and includes a plurality of operation members and the like. In the operation portion 102, the bending knob for performing bending operation of the bending portion 101B is turnably disposed, and a suction button, an air/water feeding button, switches for various functions of the endoscope, and the like are provided.

The universal cord 103 is a flexible tubular member extending from the operation portion 102. The endoscope connector 104 is a connecting member for connecting the universal cord 103 with external equipment such as a video processor and the light source apparatus.

The endoscope may be a flexible endoscope with a flexible insertion portion or a rigid endoscope with a rigid insertion portion. Further, the endoscope may be for medical use or industrial use.

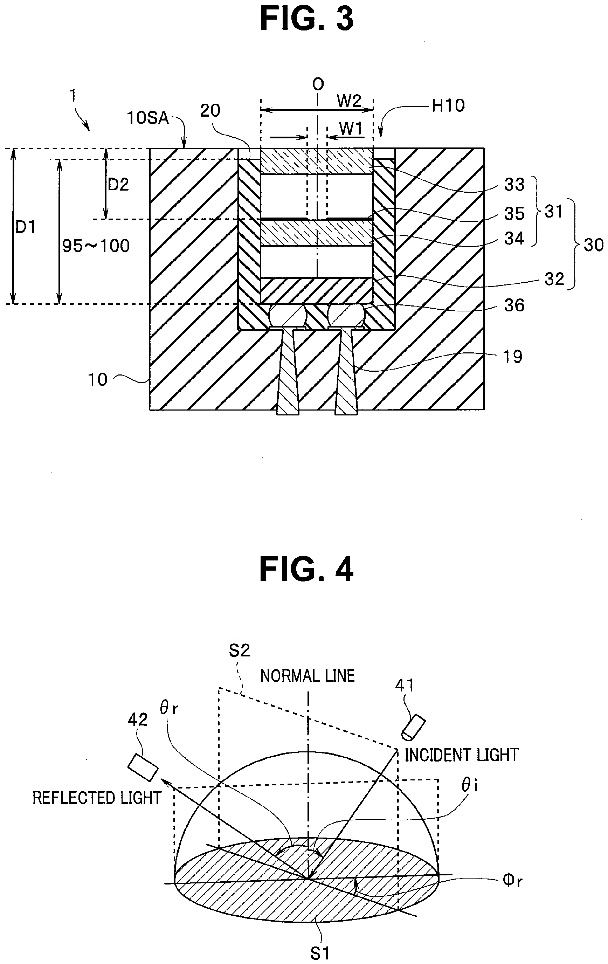

Next, a configuration of the image pickup unit 1 provided inside the distal end portion 101A will be described. Fig. 2 is a perspective view showing the configuration of the image pickup unit of the first embodiment. Fig. 3 is a cross-sectional view taken along line III-III of Fig. 2.

As shown in Fig. 2 and Fig. 3, the image pickup unit 1 includes a three-dimensional wiring board 10, resin 20, and a camera module 30.

The three-dimensional wiring board 10 is a wiring board that is not a flat board, for example, a molded interconnect device (MID), and includes a recessed portion (cavity) H10 with a bottom having an opening on an upper surface 10SA. The camera module 30 is housed in the recessed portion H10.

A base material of the three-dimensional wiring board 10 is non-conducting resin, in particular, an engineering plastic that can be molded. The base material is, for example, PA (polyamide), PC (polycarbonate), LCP (liquid crystal polymer), PEEK (polyether ether ketone), nylon, PPA (polyphthalamide), or ABS (acrylonitrile/butadiene/styrene), or composite resin of the aforementioned resins into which an inorganic filler is blended.

The resin 20 fills a gap formed between the recessed portion H10 and the camera module 30. More specifically, the resin 20 fills a gap formed between side and bottom surfaces of the recessed portion H10 and the camera module 30. The resin 20 is, for example, epoxy resin to which black carbon slurry is added and has a light shielding property. Note that the black material added to the resin 20 is not limited to carbon slurry, and may be carbon powder.

It is preferable that the resin 20 should fill a portion from the bottom surface of the recessed portion H10 in a range of 95% to 100% of an entire length D1 of the image pickup unit 1.

The camera module 30 includes a stacked lens 31 including a plurality of lenses 33, 34 and an aperture 35, and an image pickup device 32. Note that in the cross-sectional view, the plurality of lenses 33, 34 are illustrated as flat boards. A configuration of an optical system, that is, a configuration (thickness, shape), a type, the number, and a stacked order of the plurality of lenses 33, 34 and the aperture 35 can be variously modified in accordance with the specification.

It is preferable that a distance D2 from an outermost surface (front surface) of the stacked lens 31 to the aperture 35 in a direction along an optical axis O of the image pickup unit 1 should be 30% to 60% of the entire length D1 of the image pickup unit 1. Further, it is preferable that an opening diameter W1 of the aperture 35 should be 6% to 9% of a maximum dimension W2 of the stacked lens 31 in a direction perpendicular to the optical axis O of the image pickup unit 1.

The image pickup device 32 includes a light receiving portion composed of a CCD and the like. The image pickup device 32 is connected to wiring 19 of the three-dimensional wiring board 10 via an external electrode 36 such as a solder ball, for example. The image pickup device 32 receives a drive signal from the outside via the external electrode 36 and the wiring 19 and transmits an image pickup signal to the outside.

Note that in the camera module 30, a semiconductor device for processing of the image pickup signal may be stacked on a lower surface of the image pickup device 32 or a cover glass may be disposed on an upper surface of the image pickup device 32.

The resin 20 of the present embodiment has the following optical properties by containing 0.2 g to 0.4 g of carbon slurry per 100 g of the epoxy resin. Thus, the resin 20 can reduce scattered light on the surface to suppress flare.

Fig. 4 is a view for explaining a relation between incident light and reflected light.

As shown in Fig. 4, a polar angle of light (incident light) emitted from a light source 41 to a surface S1 of an object is assumed to be θi. An azimuth angle from an incident surface S2 of light reflected (reflected light) by the surface S1 is assumed to be ϕr. A polar angle of the light reflected (reflected light) by the surface S1 is assumed to be θr.

The resin 20 has properties that the transmittance and the reflectance of light (visible light) having a wavelength of 380 to 780 nm are equal to or lower than 0.5% and equal to or lower than 5%, respectively, by containing 0.2 g to 0.4 g of carbon slurry per 100 g of the epoxy resin.

Further, when θi is 45 to 75 degrees, ϕr is -60 to 60 degrees, and θr is -85 to 85 degrees, the resin 20 has a property that a value of a bidirectional reflectance distribution function (BRDF) of the reflected light detected by a detector 42 is equal to or lower than 0.1 of the incident light. Note that ϕr ≠ 0 and θi ≠ θr.

The reason why the polar angle θi in the incident direction is set at 45 to 75 degrees is that light outside the range does not affect the flare. For example, when the polar angle θi in the incident direction is equal to or greater than 75 degrees, reflection is required a plurality of times and the light outside the range is not detected as the flare. The reason why the azimuth angle ϕr from the incident surface is set at -60 to 60 degrees is that the light outside the range is shielded by the aperture 35. The reason why the polar angle θr from the incident surface is set at - 85 to 85 degrees is that the light outside the range does not affect the flare. The reason of setting ϕr ≠ 0 and θi ≠ θr is for eliminating a specular reflection.

In the image pickup unit 1 of the present embodiment, a portion between the three-dimensional wiring board 10 and the camera module 30 is filled with the resin 20 having the aforementioned optical properties. Thus, as compared to a case of filling with resin simply having a light shielding property, the image pickup unit 1 of the present embodiment can reduce the scattered light on the surface to suppress the flare.

### (Second embodiment)

Next, a second embodiment will be described.

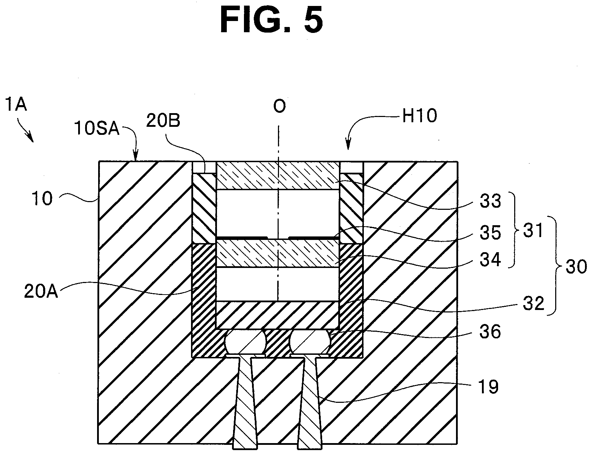

Fig. 5 is a cross-sectional view showing a configuration of an image pickup unit of the second embodiment. Note that in Fig. 5, for the same components as the components of Fig. 3, the same reference signs are assigned and the descriptions will be omitted.

As shown in Fig. 5, in an image pickup unit 1A, a gap formed between the recessed portion H10 of the three-dimensional wiring board 10 and the camera module 30 is filled with first resin 20A and second resin 20B.

The first resin 20A fills a portion from the bottom surface of the recessed portion H10 to the aperture 35 or a lower surface side relative to the aperture 35. The first resin 20A is resin having a greater dielectric breakdown resistance value and a smaller viscosity than the second resin 20B.

The second resin 20B has a dielectric breakdown voltage per 100 µm being less than 15 kV. Meanwhile, the first resin 20A has a dielectric breakdown voltage per 100 µm being equal to or greater than 15 kV. The dielectric breakdown resistance value of the first resin 20A filling the gaps of the plurality of external electrodes 36 is increased in such a manner, so that the antistatic performance can be improved.

Further, the first resin 20A has a viscosity smaller than the viscosity of the second resin 20B so as to easily fill gaps of the plurality of external electrodes 36 disposed between the recessed portion H10 and the bottom surface of the camera module 30.

The second resin 20B is the same as the resin 20 of the first embodiment, that is, 0.2 to 0.4 g of carbon slurry is contained per 100 g of epoxy resin. The second resin 20B fills a portion from the aperture 35 or a lower surface side relative to the aperture 35 to the outermost surface of the stacked lens 31, more specifically, in a range of 95% to 100% of the entire length D1 of the image pickup unit 1. The second resin 20B is the same as the resin 20 of the first embodiment, and can thus reduce the scattered light on the surface to suppress the flare.

The image pickup unit 1A of the present embodiment houses the camera module 30 in the recessed portion H10 of the three-dimensional wiring board 10 and connects the wiring 19 of the three-dimensional wiring board 10 and the camera module 30 via the external electrodes 36.

Subsequently, the first resin 20A fills a gap between the recessed portion H10 of the three-dimensional wiring board 10 and the camera module 30, from the bottom surface of the recessed portion H10 to the position of the aperture 35 (or the lower surface side relative to the aperture 35).

Finally, the second resin 20B fills the gap between the recessed portion H10 of the three-dimensional wiring board 10 and the camera module 30, from the aperture 35 (or the lower surface side relative to the aperture 35) to the outermost surface of the stacked lens 31, so that the image pickup unit 1A can be assembled.

The present invention is not limited to the aforementioned embodiments, and various changes, modifications, and the like are available within the range without changing the gist of the present invention.

## Claims

1. An image pickup unit comprising:
a three-dimensional wiring board including a recessed portion having a side surface and a bottom surface;
a camera module disposed in the recessed portion; and
resin having a light shielding property that fills a gap formed between the side surface and the camera module and between the bottom surface and the camera module, wherein
in the resin, a transmittance and a reflectance of light having a wavelength of 380 to 780 nm are equal to or lower than 0.5% and equal to or lower than 5%, respectively, and
when a value of a bidirectional reflectance distribution function in a direction in which an azimuth angle from an incident surface is ϕr and a polar angle is θr, with a polar angle in an incident direction as θi is BRDF (θi, ϕr, θr), in a case where θi is 45 to 75 degrees, ϕr is -60 to 60 degrees, and θr is -85 to 85 degrees, the value of the bidirectional reflectance distribution function is equal to or lower than 0.1 of incident light, wherein, ϕr ≠ 0 and θi ≠ θr.

2. The image pickup unit according to claim 1, wherein
the camera module comprises:
a stacked lens including a plurality of lenses and an aperture; and
an image pickup device, and
a distance from an outermost surface of the stacked lens to the aperture in a direction along an optical axis of the camera module is 0.3 to 0.7 of an entire length of the camera module.

3. The image pickup unit according to claim 2, wherein an opening diameter of the aperture is 0.06 to 0.09 of a maximum dimension of the stacked lens in a direction perpendicular to the optical axis of the camera module.

4. The image pickup unit according to claim 1, wherein the resin is epoxy resin to which black carbon slurry is added.

5. The image pickup unit according to claim 2, wherein
the resin comprises first resin that fills a bottom surface side of the recessed portion and second resin that fills an opening side of the recessed portion,
the first resin fills a portion from the bottom surface of the recessed portion to a position where the aperture is provided, the first resin having a dielectric breakdown resistance value greater than a dielectric breakdown resistance value of the second resin, and
the second resin fills a portion from the aperture to a position of the outermost surface of the stacked lens.

6. An endoscope comprising:
an image pickup unit at a distal end of an insertion portion inserted into a subject, the image pickup unit including:
a three-dimensional wiring board including a recessed portion having a side surface and a bottom surface;
a camera module disposed in the recessed portion; and
resin having a light shielding property that fills a gap formed between the side surface and the camera module and between the bottom surface and the camera module, wherein
in the resin, a transmittance and a reflectance of light having a wavelength of 380 to 780 nm are equal to or lower than 0.5% and equal to or lower than 5%, respectively, and
when a value of a bidirectional reflectance distribution function in a direction in which an azimuth angle from an incident surface is ϕr and a polar angle is θr, with a polar angle in an incident direction as θi is BRDF (θi, ϕr, θr), in a case where θi is 45 to 75 degrees, ϕr is -60 to 60 degrees, and θr is -85 to 85 degrees, the value of the bidirectional reflectance distribution function is equal to or lower than 0.1 of incident light, wherein, ϕr ≠ 0 and θi ≠ θr.

7. The endoscope according to claim 6, wherein
the camera module comprises:
a stacked lens including a plurality of lenses and an aperture; and
an image pickup device, and
a distance from an outermost surface of the stacked lens to the aperture in a direction along an optical axis of the camera module is 0.3 to 0.7 of an entire length of the camera module.

8. The endoscope according to claim 7, wherein an opening diameter of the aperture is 0.06 to 0.09 of a maximum dimension of the stacked lens in a direction perpendicular to the optical axis of the camera module.

9. The endoscope according to claim 6, wherein the resin is epoxy resin to which black carbon slurry is added.

10. The endoscope according to claim 7, wherein
the resin comprises first resin that fills a bottom surface side of the recessed portion and second resin that fills an opening side of the recessed portion,
the first resin fills a portion from the bottom surface of the recessed portion to a position where the aperture is provided, the first resin having a dielectric breakdown resistance value greater than a dielectric breakdown resistance value of the second resin, and
the second resin fills a portion from the aperture to a position of the outermost surface of the stacked lens.

11. A method for manufacturing an image pickup unit, the method comprising steps of:
connecting wiring of a three-dimensional wiring board and a camera module via an external electrode, with the camera module housed in a recessed portion of the three-dimensional wiring board;
filling, with first resin, a gap between the recessed portion of the three-dimensional wiring board and the camera module, from a bottom surface of the recessed portion to a position of an aperture of the camera module; and
filing, with second resin, a gap between the recessed portion of the three-dimensional wiring board and the camera module, from the aperture to an outermost surface of a stacked lens of the camera module,
wherein the first resin is resin having a greater dielectric breakdown resistance value and a smaller viscosity than the second resin.
